# EUROPEAN PATENT APPLICATION

(11) **EP 2 053 402 A1**
(43) Date of publication of application: **29.04.2009**
(21) Application number: 09153734.0
(22) Date of filing: 26.01.2007
(51) Int. Cl.: G01N 33/58, C12Q 1/68, C09K 11/59, C12N 15/09, G01N 21/78, C12M 1/00

(54) **Fluorescent marker for living organism and fluorescent marking method for the same**

(30) Priority: 27.01.2006 JP 2006019458; 27.01.2006 JP 2006019459
(62) Divisional of application: 07707487.0
(71) Applicant: Konica Minolta Medical & Graphic, Inc., Hino-shi, Tokyo 191-8511 (JP)
(72) Inventor: Tsukada, Kazuya, Hino-shi Tokyo 191-8511 (JP); Goan, Kazuyoshi, Hino-shi Tokyo 191-8511 (JP); Furusawa, Naoko, Hino-shi Tokyo 191-8511 (JP); Hoshino, Hideki, Hino-shi Tokyo 191-8511 (JP)
(74) Representative: Henkel, Feiler & Hänzel

(57) **Abstract**

A fluorescent label for a biomaterial which comprises inorganic fluorescent nanoparticles characterized by having on a surface of the inorganic fluorescent nanoparticles, a modifying group containing a living organism binding portion capable of specifically binding to a living organism; and a link forming modifying group which combines the inorganic fluorescent nanoparticles together. Use of this fluorescent label makes it possible to conduct an analysis at an elevated sensitivity and an elevated accuracy in, for example, analyzing a biomaterial such as detecting a DNA or an RNA by the hybridization method or determining the base sequence of a DNA, detecting an antigen by the ELISA method and so on.

## Description

### TECHNICAL FIELD

The present invention relates to a fluorescent marker for a living organism, a fluorescent marking method of a living organism, and a bioassay method employing these.

### BACKGROUND

In view of bioinformatics and proteomics, in order to analyze functions and kinetics of mRNA and proteins in living organisms, proposed is a method concept designated as so-called "single-molecule imaging", in which genome functions, manifestation control of genes, or behaviors of each living organism molecule are seized at the molecular level. Based on this, research and development of single-molecule imaging technologies has been conducted which intends to clarify causes of illness and functions of medicines. In the above single-molecule imaging technology, one molecule of a fluorescent marker binds to one molecule of a targeted living organism (being a protein, such as an antibody, DNA, RNA, or oligonucleotide). The resulting product is exposed to the specified excitation light, followed by detection of luminescence from the fluorescent markers, whereby it is tried to collect information of the kinetics of living organisms. When the luminescent lifetime of a fluorescent marker is relatively short and discoloration is relatively rapid, or when the intensity of the resulting luminescence is insufficient, it is obvious that it is not possible to achieve sufficient observation.

Organic fluorescent dyes, which have commonly been employed to analyze living organisms, for example, those utilizing bioluminescence employing a reaction of luciferin, being a fluorescent protein, with luciferase, or luminescent molecules, are insufficient in the above required luminescent lifetime and luminescent intensity. Consequently, in the field of single-molecule imaging technology, fluorescent markers employing inorganic fluorescent nanoparticles are being developed, being those which will replace conventional organic fluorescent dyes. Inorganic fluorescent nanoparticles exhibit advantages compared to organic fluorescent dyes in such a manner that the emission spectra are sharper (narrow half band width), the luminescent intensity is stronger, the detection accuracy is higher due to the relatively large difference between the excitation wavelength and the emission wavelength, the excitation wavelength is more readily controlled in response to particle size, and the fluorescent lifetime is longer.

In regard to such a fluorescent marker employing inorganic fluorescent nanoparticles in the field of single-molecule imaging technology, in addition to the above items, binding capability to targeted living organisms, namely specificity or binding force is also highly valuated. However, in fluorescent markers (refer, for example, to Patent Document 1) utilizing conventional inorganic fluorescent nanoparticles, no study has been conducted to improve binding capability with regard to a modifying group from the aspect of steric hindrance which significantly relates to the above binding capability. Further, with regard to fluorescent markers utilizing inorganic fluorescent nanoparticles, in order to enhance detection accuracy, an increase in luminescent intensity is being pursued. Conventionally, experiments (refer, for example, to Patent Document 1) to increase the luminescent intensity have been carried out from the aspect of particle structures such as diameter, materials or a core/shell structure of individual inorganic fluorescent nanoparticles. However, room for more improvement still remains. (Patent Document 1) Japanese Patent Publication Open to Public Inspection JP-A No. 2005-172429

### DISCLOSURE OF THE INVENTION

### PROBLEMS TO BE SOLVED BY THE INVENTION

An object of the present invention is to provide a fluorescent marker incorporating inorganic fluorescent nanoparticles, which exhibits enhanced binding capability to living organisms to realize more appropriate marking, and a fluorescent marking method for living organisms.

### PROBLEMS TO SOLVE THE PROBLEMS

The above-described object can be achieved by the following embodiments.
1. A fluorescent marker for a living organism comprising inorganic fluorescent nanoparticles having on a surface of the nanoparticles a modifying group containing a reactive portion,
   wherein the reactive portion is located at a most remote site of the modifying group from the surface of the nanoparticles; the reactive portion comprises at least one selected from the group consisting of bases consisting of DNA or RNA, nucleotides, polynucleotides and intercalators; and the reactive portion is capable of specifically binding to a living organism.
2. The fluorescent marker for a living organism of item 1,
   wherein the inorganic fluorescent nanoparticle is a spherical semiconductor nanoparticle having an average particle diameter of 1-10 nm.
3. The fluorescent marker for a living organism of item 2,
   wherein the inorganic fluorescent nanoparticle has a core-shell structure comprising:
   a core of a semiconductor; and
   a shell having a different composition from the core.
4. The fluorescent marker for a living organism of item 3,
   wherein the inorganic fluorescent nanoparticle has:
   the core of Si; and
   the shell of SiO₂.
5. The fluorescent marker for a living organism of any one of items 1 to 4, wherein the inorganic fluorescent nanoparticles comprise a link forming modifying group which combines the inorganic fluorescent nanoparticles together.
6. A fluorescent marker for a living organism comprising inorganic fluorescent nanoparticles having on a surface of the nanoparticles:
   a modifying group containing a living organism binding portion capable of specifically binding to a living organism; and
   a link forming modifying group which combines the inorganic fluorescent nanoparticles together.
7. The fluorescent marker for a living organism of item 6,
   wherein a surface covering ratio of the link forming modifying groups on the surfaces of the inorganic fluorescent nanoparticles is 10-50%.
8. The fluorescent marker for a living organism of items 6 or 7, wherein the inorganic fluorescent nanoparticle is a spherical particle having an average particle diameter of 1-10 nm.
9. The fluorescent marker for a living organism of any one of items 6 to 8, wherein the inorganic fluorescent nanoparticle has a core-shell structure comprising:
   a core of a semiconductor; and
   a shell having a different composition from the core.
10. The fluorescent marker for a living organism of item 9,
   wherein the inorganic fluorescent nanoparticle has:
   the core of Si; and
   the shell of SiO₂.
11. The fluorescent marker for a living organism of any one of items 6 to 10, wherein the link forming modifying group combines the inorganic fluorescent nanoparticles together thorough a hydrogen bond, an ionic bond, a covalent bond or a Van der Waals' force.
12. The fluorescent marker for a living organism of any one of items 6 - 11, wherein the inorganic fluorescent nanoparticles are in a form of a cluster having a particle number of 2-20.
13. A method of fluorescent marking for a living organism using the fluorescent marker of any one of items 1 to 12, the method comprising the step of:
   binding the living organism with the inorganic fluorescent nanoparticle through the reactive portion of the modifying group capable of specifically binding to the living organism.

The inventors of the present invention achieved the present invention based on the following findings. By introducing, onto the surface of the above inorganic fluorescent nanoparticles, a modifying group having a reactive portion located, at the most remote site, which specifically binds the nanoparticle to a living organism selected from the group consisting of bases constituting DNA or RNA, nucleotides or polynucleotides, and intercalators, problems due to the steric hindrance are avoided, whereby it is possible to prepare fluorescent markers which exhibit enhanced binding capability to living organisms to be analyzed. Hereinafter, the modifying group having a portion which binds to a living organism is also simply called as a living organism binding modifying group.

In the present invention, it is preferable that the above inorganic fluorescent nanoparticles are spherical at a diameter of 1 - 10 nm. Further, it is more preferable that the above inorganic fluorescent nanoparticles exhibit a core-shell structure composed of a semiconductor core and a shell in the form of layer, which is different from the above core in compositions, and inorganic fluorescent nanoparticles are particularly preferred in which Si is employed as the core and SiO₂ is employed as the shell.

Via the modifying group as described above, it is possible to appropriately bind the fluorescent markers to living organisms.

Further, the inventors of the present invention achieved the present invention based on the following findings. Modifying groups to link the above fluorescent markers to each other are introduced onto the surface of inorganic fluorescent nanoparticles employed as a fluorescent marker, and by forming a cluster in which the above fluorescent markers are bound to each other, luminescent intensity of a material to be detected is still more enhanced.

The inorganic fluorescent nanoparticles of the present invention described in item 6 have, on the surface, a modifying group (being a living organism binding modifying group) and another modifying group (being a link forming modifying group) to link the above inorganic fluorescent nanoparticles to each other. Inorganic fluorescent nanoparticles are linked to each other due to formation of a hydrogen bond, an ionic bond, or a covalent bond via the above link forming modifying group, or due to van der Waals' force.

The surface coverage ratio of inorganic fluorescent nanoparticle via molecules forming the above link forming modifying group is preferably 10 - 50%. Further, the above inorganic fluorescent nanoparticles link to each other to form inorganic fluorescent nanoparticle clusters, each of which is composed of 2 - 20 particles. It is preferable that the number of particles is regulated via temperature or pH.

In the present invention described in item 6, it is preferable that the above inorganic fluorescent nanoparticles are spherical at a diameter of 1 - 10 nm. Further, it is more preferable that the above inorganic fluorescent nanoparticles exhibit a core-shell structure composed of a semiconductor core and a shell in the form of layer, which is different from the above core in composition, and inorganic fluorescent nanoparticles are particularly preferred in which Si is employed as the core and SiO₂ is employed as the shell.

### EFFECTS OF THE INVENTION

According to the present invention, in detection of DNA and RNA employing a hybrid formation method, an analysis of living organisms such as determination of the base sequence of DNA, or an analysis to detect an antigen, employing the ELISA method, binding capability of fluorescent markers is enhanced, whereby it is possible to perform highly sensitive and accurate analysis employing the single-molecule imaging technology.

### PREFERRED EMBODIMENTS TO CARRY OUT THE INVENTION

(Inorganic Fluorescent Nanoparticles) Inorganic fluorescent nanoparticles employed in the fluorescent markers of the present invention are fluorescent materials which are capable of resulting in luminescent intensity higher than those having the same bulk, via spatially trapping excitons in a particle in nanometer (nm) order, being a so-called quantum trapping effect. It is common knowledge in the art that the luminescent intensity and fluorescent wavelength of the above nanoparticles vary depending on the particle diameter, whereby it is possible to prepare those of the desired particle diameter. In the preset invention, in view of emission of suitable fluorescence for analysis employing the single-molecule imaging, it is preferable that inorganic fluorescent nanoparticles are spherical or nearly spherical semiconductor nanoparticles of which diameter is commonly 0.5 - 20 nm, is preferably 1 - 10 nm, but is more preferably 2 - 5 nm. It is possible to determine such a particle diameter via observation employing a TEM (being a transmission type electron microscope). An average value is to be employed, which is obtained by observing at least 200 particle images.

Further, in the present invention, it is preferable that the above inorganic fluorescent nanoparticles are those which have a so-called core-shell structure composed of a semiconductor core and a shell in the form of a layer, which is different from the above core in composition. By employing, as a shell, a material having a band gap which is greater than that of a material to be employed as a core, the quantum trapping effect is stabilized, whereby it is possible to more enhance luminescence intensity compared to inorganic fluorescent nanoparticles of the same size having no core-shell structure. "Diameter of inorganic fluorescent nanoparticles having such a core-shell structure" refers to a particle diameter including the shell layer portion.

Semiconductor nanoparticles, which constitute the above inorganic fluorescent nanoparticles, are not particularly limited. Examples include semiconductors composed of Group I - VII compounds such as CuCl, semiconductors composed of Group II - VI compounds such as CdS or SdSe, semiconductors composed of Group III - V compounds such as InS, and IV Group semiconductors such as Ge, as well as crystals thereof. Of these, any appropriate semiconductors may be selected and then employed. Further, in regard to the above core-shell structure, any appropriate combination such as CdSe as a core/ZnS as a shell, or Si as a core/SiO₂ as a shell may be selected depending on employed semiconductor nanoparticles. In the present invention, it is appropriate to employ semiconductor nanoparticles having the core-shell structure of Si as a core/SiO₂ as a shell or Ge as a core/GeO₂ as a shell since materials which are concerned with environmental pollution and toxicity to operators are not employed, while sufficient luminescence is obtained.

(Modifying Group in Item 1) The fluorescent markers of the present invention are those in which a specified "modifying group" is introduced onto the surface of the inorganic fluorescent nanoparticles, described as above. The above modifying group is composed, for example, of a reactive portion (hereinafter referred to as a "living organism binding portion") capable of specifically binding to living organisms, a portion (hereinafter referred to as a "surface binding portion") which directly bind to the surface of the inorganic fluorescent nanoparticle, and an intermediate portion (hereinafter referred to as a "spacer") which links the above living organism binding portion to the above surface binding portion. It is possible for the above fluorescent nanoparticle to bind to a living organism to be marked via the above modifying group.

Examples of the above living organism binding portion include bases constituting DNA or RNA, such as adenine, guanine, cytosine, thymine, or uracyl; polynucleotides (including oligonucleotides) in which a single nucleotide or a plurality of nucleotides are joined; and intercalators.
Any of these compounds are capable of specifically binding to the targeted living organism (being the gene portion). The above base constituting DNA or RNA binds to a base complementary corresponding to each of them, and the above nucleotide or polynucleotide binds to nucleotide or polynucleotide having a complimentary base sequence for each of them. The above polynucleotide incorporates a plurality of bases, whereby it is possible to form a more stabilized bond compared to the case in which a single base constitutes DNA or RNA.

Further, the above intercalators are compounds, each of which is inserted between paired ds (double strand) DNA bases to form bonds. Employed as intercalates used in the present invention may, for example, be compounds having a heterocyclic structure such as ethydium bromide or acridine which is also employed as a fluorescent agent. Such an intercalator is inserted between paired bases to form bonds during formation of dsDNA. Consequently, for example, it is possible to employ, as a fluorescent marker, to detect dsDNA in the hybrid forming method.

The above living organism binding portion in the modifying group is located at the most remote site from the surface of an inorganic fluorescent nanoparticle. Due to that, the inorganic fluorescent nanoparticles bind to targeted living organisms without causing steric hindrance, whereby it is possible to mark them.

For example, when a modifying group is prepared by introducing the living organism binding portion into a compound of a rigid and linear molecular structure, by binding the living organism binding portion to the terminals of the above compound, it is possible to locate it at the most remote site from the surface of the inorganic fluorescent nanoparticle. Further, as described below, when the living organism binding portion is introduced into a long chain compound having a structure such as ethylene glycol, a reactive functional group is positioned on the carbon atom which is located at the most remote site from the surface of the inorganic nanoparticle and the living organism binding portion may undergo binding via the above functional group.

It is possible to confirm the presence of the living organism binding portion in the position which is located at the most remote site from the surface of the inorganic fluorescent nanoparticle of the modifying group by preparing a calibration curve of the introduction position of the living organism binding portion with respect to the binding capability of the living organism. For example, a modifying group is introduced into an inorganic fluorescent nanoparticles, employing a compound which allows a reactive functional group to position itself on each of carbon atoms of a long chain molecular structure, and each of the fluorescent markers is bound to a living organism, followed by determination of the resulting fluorescent intensity. During the determination, a modifying group employed in the fluorescent marker which is subjected to detection of the highest intensity of fluorescence is one which is located at the most remote site from the surface of the inorganic fluorescent nanoparticle.

If desired, the fluorescent markers of the present invention may have modifying groups, other than those employed above, such as a phosphoric acid group, an amino group, or a polyoxyethylene group to enhance hydrophilicity in the amount range which does not adversely affect the targeted effects of the present invention.

(Modifying Group in Item 6) The fluorescent marker of the present invention described in item 6 is one in which at least each one of two types of "modifying groups" are introduced into the inorganic fluorescent nanoparticle, described as above. One of the modifying groups of the present invention is one capable of specifically binding to living organisms and is designated as a "living organism binding portion". Another modifying group of the present invention is one which links the fluorescent markers of the present invention to each other, and is designated as a "link forming modifying group". Further, if appropriate, the fluorescent marker of the present invention may have other modifying groups in the amount range which does not adversely affect the targeted effects of the present invention.

The surface coverage ratio of molecules forming the above link forming modifying group on the inorganic fluorescent nanoparticle is preferably 10 - 50%, in view of ease of attaining preferred cluster formation, but is more preferably 15 - 40%. "Surface coverage ratio", as described in the present invention, refers to the ratio of the area covered with molecules, which form the link forming modifying group, to the total surface area of the inorganic fluorescent nanoparticle. It is possible to determine the above ratio via observation employing TEM.

The above living organism binding portion which corresponds to the use of the fluorescent marking in the targeted analysis may be introduced via any of the conventional methods, and the embodiments are not particularly limited. For example, employed as the living organism binding portion of the present invention may be a modifying group, in which ss (single strand) DNA is bound, which is employed to detect DNA via the hybrid formation method, a modifying group bound to biotin or avidin which is employed to detect protein via the ELISA method, and a modifying group bound to lectin or specific antibody, which is employed to detect cancer cells.

Employed as the above link forming modifying group may be compounds capable of being linked to each other via a hydrogen bond, an ionic bond, a covalent bond, or van der Waals' force. For example, listed as the above covalent bond are a disulfide bond between thiol groups and a peptide bond between an amino group and carboxyl group, and compounds having any of these functional groups may be introduced onto the surface of the inorganic fluorescent nanoparticle. Further, it is possible to prepare the link forming modifying group in such a manner that by employing a nucleotide chain, inorganic fluorescent particles having the complementary nucleotide chain are linked to each other.

The number of reactive portions which form the above hydrogen bond, ionic bond, or covalent bond is not particularly limited and is capable of being appropriately controllable. For example, the link forming modifying group may be formed via one covalent bond exhibiting relatively strong binding force or via a plurality of hydrogen bonds exhibiting relatively weak binding force.

In the present invention, as long as the link forming modifying groups are linkable to each other, they may be employed individually or in combinations of a plurality of types. For example, in one embodiment of the present invention, it is possible to introduce, into all fluorescent markers, an identical modifying group having a thiol group. Further, the present invention includes an embodiment in which two types of link forming modifying groups such as a link forming modifying group having an amino group and a link forming modifying group having a carboxyl group are introduced into one fluorescent marker. Still further, in another embodiment of the present invention, it is possible to employ link forming modifying groups in such a manner that a fluorescent marker, into which identical or different modifying groups having an amino group are introduced, is combined with a fluorescent marker into which identical or different modifying groups having a carboxyl group are introduced so that links are formed via a peptide bond. It is preferable that functional groups existing in the identical fluorescent marker do not undergo binding with each other so that link formation among fluorescent markers is not adversely affected.

The above living organism binding portions and link forming modifying groups are each commonly introduced onto the surface of the inorganic fluorescent nanoparticle as a different molecule. However, as one embodiment of the present invention, it is possible to introduce, onto the surface of the inorganic fluorescent nanoparticle, a group composed of a living organism binding portion and a link forming modifying group in one molecule having a branched structure.

In the present invention, the combined embodiment of item 1 and item 6, namely, the embodiment of item 5 is a preferred embodiment to achieve the marked effects of the present invention.

(Production Method of Fluorescent Markers: Preparation of Inorganic Fluorescent Nanoparticles) It is possible to produce the targeted inorganic fluorescent nanoparticles used in the present invention, employing methods known in the art. Production methods are not particularly limited, and examples include a gas phase method such as a CVD method, a laser ablation method, a silane decomposition method, or a Si electrode vaporization method, as well as a liquid phase method such as an electrolysis method, or a reverse micelle method. Inorganic fluorescent nanoparticles produced via these methods are occasionally present in a suspension dispersed state in a liquid or in a state fixed on a plate. However, states are not particularly limited as long as they are in such a state in which it is possible to introduce the modifying group as described below.

More specifically, an amorphous SiO₂ membrane is built up on a Si substrate employing, for example, a plasma CVD method or a sputtering method, and annealed at a relatively high temperature so that Si nanoparticles are formed in the SiO₂ membrane. Subsequently by dissolving the SiO₂ membrane employing HF, aggregated nanoparticles are obtained on the liquid surface. Further, the surface undergoes natural oxidation under an oxygen environment or is heated to undergo thermal oxidation, whereby the targeted inorganic fluorescent nanoparticles are prepared in the structure of Si as a core/SiO₂ as a shell.

(Introduction of Modifying Group) The modifying group of the present invention may undergo introduction in such a manner that by employing compounds which are bondable onto the surface of the inorganic fluorescent nanoparticle, the above surface binding portions are formed and the resulting compound is bound to the above living organism binding portion forming compound. Further, after binding a surface binding portion forming compound to a spacer forming compound, the above living organism binding portion forming material may be bound.

Employed as the above surface binding portion forming compounds may, for example, be silane coupling agents which are widely used to bind inorganic materials to organic materials. The above silane coupling agents are compounds which have, at one end of the molecule, an ethoxy group or a methoxy group which results in a silanol group (SiOH) via hydrolysis, and at the other end, a functional group such as a thiol group (being a mercapto group), an amino group, an epoxy group (being a glycidyl group), or an aldehyde group, and bind to inorganic materials via the oxygen atom of the above silanol group. For example, mercaptopropyltriethoxysilane is cited as a conventionally employed silane coupling agent.

It is possible to bind the silane coupling agent to the fluorescent nanoparticle via oxygen employing common methods known in the art. For example, the following procedures are feasible. Initially, inorganic fluorescent nanoparticles in the structure of Si as a core/SiO₂ as a shell are prepared, followed by dispersion into hydrogen peroxide, whereby the shell surface undergoes hydroxylation. Subsequently, the solvent is replaced with toluene and mercaptopropyltriethoxysilane is added, followed by reaction over two hours, whereby mercaptopropylethoxysilane is introduced onto the shell surface.

Further, instead of the above silane coupling agents, it is possible to form a surface binding portion employing dimercaptosuccinic acid or 11-mercaptoundecanic acid. For example, when semiconductor nanoparticles are produced employing the reverse micelle method, described below, the surface is covered with TOPO (tri-n-octylphosphine chloride), which is capable of undergoing substitution with dimercaptosuccinic acid or 11-mercaptoundecanic acid. By doing so, dimercaptosuccinic acid or 11-mercaptoundexanic acid is bound to the surface of the inorganic fluorescent nanoparticle via a sulfur atom. However, since the other end has a carboxyl group, it is possible to bind the compounds to form a living organism binding portion, as described below.

Further, in the present invention, an embodiment is acceptable in which a compound which directly binds to inorganic fluorescent nanoparticles, a compound which forms a living organism binding portion and/or a link forming modifying group, and a compound, such as a silane coupling agent, which forms a surface binding portion, are bound via an intermediate portion (hereinafter also referred to as a "spacer"). Namely, the above spacer is a compound which incorporates a portion capable of binding a compound which directly binds inorganic fluorescent nanoparticles and a portion capable of binding a compound to form a living organism binding portion and/or a link forming modifying group. As such a compound, it is possible to employ organic molecules, such as sulfo-SMCC (maleimidomethylcyclohexanecarboxylic acid sulfohydroxysuccinimide ester sodium salt), which are called a bifunctional cross-linker.

For example, above sulfo-SMCC has two reactive portions exhibiting directivity toward an amino group or a thiol group and can be employed as a compound in which, for example, one is bound to a silane coupling agent and the other is bound to the compound which forms a living organism binding portion. Further, employed as the bifunctional cross-linker may be compounds in such a structure in which, a functional group capable of binding a material which forms a surface binding portion and a functional group capable of binding a material which forms a living organism binding portion are introduced at both ends of oxyalkylene such as polyethylene glycol (PEG).

On the other hand, by previously introducing, via a conventional method, a functional group capable of binding a surface binding portion or a functional group of a compound which forms a spacer into a portion of, for example, a base constituting DNA or RNA, polynucleotide, or an intercalator, capable of binding to the targeted living organs, it is possible to bind the living organism binding portion to any of the above compounds. For example, when a silane coupling agent having a thiol group is allowed to react with nucleotide also having a thiol group, nucleotide is introduced into a modifying group via formation of a disulfide bond.

Further, it is possible to carry out introduction of the link forming modifying group, employing compounds having at least two reactive functional groups such as a thiol group, an amino group, or a carboxyl group. Of these functional groups, one is employed to link to each other via a hydrogen bond, an ionic bond, a covalent bond, or van der Waals' force. The other is employed to bind a functional group of the compound forming a surface binding portion or a spacer. Examples of compounds which form such a link forming modifying group include dicarboxylic acids and amino acids having at least an amino group and a carboxyl group.

In the present invention, when the above living organism binding portion and the above link forming modifying group are each introduced onto the surface of the inorganic fluorescent nanoparticle as a different molecule, it is possible to regulate the surface coverage ratio of each molecule employing, for example, a method in which a mixture of two different silane coupling agents is employed. For example, in the above method, a silane coupling agent capable of only binding inorganic fluorescent nanoparticles, and a compound which forms a living organism binding portion and a silane coupling agent capable of binding inorganic fluorescent nanoparticles and a compound which forms the link forming modifying group, are blended at a ratio which is the same as the targeted surface coverage ratio, and a mixed solution incorporating these two silane coupling agents may be allowed to react with inorganic fluorescent nanoparticles, and subsequently, may also be allowed to react with a solution incorporating the living organism binding portion and with the link forming modifying group. It is possible to control the surface modification ratio in an equilibrium state by selecting a binding portion depending on the order of the bonding force.

(Clusters) In the fluorescent marker of the present invention, inorganic fluorescent nanoparticles link to each other via the aforesaid link forming modifying group to form clusters. It is preferable that it is possible to regulate the number of inorganic fluorescent nanoparticles to form a cluster, via temperature or pH. It is possible to regulate bonding force among the link forming modifying group by, for example, changing the ambience such as temperature or pH, or controlling the number of modifying groups, which are introduced to inhibit the combination of the link forming modifying groups. Further, for example, when oligonucletide is employed as a link forming modifying group, it is possible to regulate the bonding force of the link forming modifying group via the co-presence of the complimentary base sequence and non-complementary base sequence.

Further, it is possible to appropriately regulate the number of inorganic fluorescent nanoparticles to form the above clusters. In order to achieve sufficient enhancement in the luminescent intensity, and to not inhibit migration in the living organism, the number is preferably 2 - 20. It is possible to confirm the number of nanoparticles in a cluster via TEM (being a transmission electron microscope) observation.

By forming a cluster in an embodiment in which inorganic fluorescent nanoparticles, which bind to the targeted living organism, link with inorganic fluorescent nanoparticles which do not directly bind to the living organism, it is possible that one molecule of the living organism is marked with more inorganic fluorescent nanoparticles than before.

It is possible to appropriately employ the above fluorescent marker of the present invention in various analyses using conventional fluorescent markers. Examples of such analyses include immunity dyeing employing a fixed cell, real time tracking of receptor-ligands, and single-molecule imaging. In these analyses, the cluster formed employing the fluorescent marker of the present invention emits more intense fluorescence, compared to the case in which the fluorescent marker is employed alone, whereby it is possibly to enhance the detection accuracy of living organisms.

(Analytical Method Employing Fluorescent Markers) The fluorescent markers of the present invention can be employed for various analyses of living organisms which carry gene information of DNA or RNA, and are specifically suitable for the following analyses.

For example, four types of fluorescent markers, emitting fluorescence at different wavelengths, in which adenine, guanine, cytosine, and thymine, or nucleotides having each of the bases are introduced into living organism binding portions, are bound to each of the ssDNA or nucleotides having the corresponding base. By employing these four types of binding markers, a fluorescent marker is bound to each base of ssDNA of unknown base sequence and subsequently, fluorescence sequence is read via exposure of excitation light, whereby it is possible to determine the base sequence of ssDNA. Further, ssDNA of an unknown base sequence is subjected to one by one molecule cutting employing an enzyme exhibiting 3' → 5' exonuclease active or 5' → 3' exonuclease active, and each of continually cut nucleotide molecules is bound to any of the above four types of fluorescent markers and the fluorescence emitted by the bound fluorescent marker is continually detected employing a highly sensitive fluorescence detector, whereby it is possible to determine the base sequence of ssDNA.

### EXAMPLES

Example 1 (Preparation of Inorganic Fluorescent Nanoparticles via the HF Etching Method) SiOₓ (x = 1.999) cast onto a silicone wafer via plasma CVD was annealed at 1,100 °C for one hour under an inert gas atmosphere, whereby Si crystals were deposited in a SiO₂ membrane. Subsequently, the resulting silicone wafer was treated at room temperature with a 1% aqueous hydrofluoric acid solution to remove the SiO₂ membrane, and Si crystals at a size of several nm, which aggregated on the liquid surface were recovered. Further, via the above hydrofluoric acid treatment, the dangling bond (being a non-bonding means) of Si atoms of the crystal surface is terminated by hydrogen, whereby Si crystals are stabilized. Thereafter, the surface of recovered Si crystals underwent natural oxidation under an oxygen atmosphere, whereby a shell layer composed of SiO₂ was formed on the periphery of the core composed of Si crystals.

(Preparation of Inorganic Fluorescent Nanoparticles via the Anodic Oxidation Method) In a solution prepared by blending hydrofluoric acid (46%), menthol (100%), and hydrogen peroxide water (30%) at a ratio of 1 : 2 : 2, electricity was applied to a counter electrode composed of p type silicone wafer and platinum at 320 mA/cm² for about one hour, whereby Si crystals were deposited. The surface of Si crystals prepared as above underwent natural oxidation under an oxygen atmosphere, whereby a shell layer composed of SiO₂ was formed on the periphery of the core composed of Si crystals.

The average diameter of all the inorganic fluorescent nanoparticles in the structure of a core as Si/a shell as SiO₂ prepared as above was 3.5 mm.

(Introduction of Oligonucleotide Terminal Modifying Group: Samples A and C) Initially, inorganic fluorescent nanoparticles prepared via the HF etching method were dispersed into 30% H₂O₂ over 10 minutes, whereby the crystal surface was hydroxidized. Subsequently, the solvent was replaced with toluene, and mercaptopropyltriethoxysilane was added in an amount of 2% of toluene, whereby SiO₂ of the uppermost layer of the inorganic fluorescent nanoparticle was silanized and was simultaneously subjected to introduction of a mercapto group. Subsequently, the solvent was replaced with pure water and buffering salts were added. Further, oligonucleotide, at one end of which a mercapto group was introduced, was added to attain 100 Nm, and the resulting mixture was allowed to stand for one hour so that inorganic fluorescent nanoparticles bind to the inorganic fluorescent nanoparticles. The fluorescent marker, prepared as above, was designated as Sample A.

Further, a fluorescent marker was prepared employing the same method described as above, except that the inorganic fluorescent nanoparticles prepared via the HF etching method were replaced with ones prepared via the anodic oxidation method. The resulting material was designated as Sample C.

(Introduction of a modifying Group which places oligonucleotide in the intermediate position: Samples B and D) Initially, inorganic fluorescent nanoparticles, prepared via the HF etching method, were dispersed into 30% H₂O₂ over 10 minutes so that the crystal surface was hydroxidized. Subsequently, the solvent was replaced with toluene, and mercaptoethyltriethoxysilane was added in an amount of 2% of toluene, and SiO₂ of the uppermost surface of the inorganic fluorescent nanoparticles was silanized over two hours, while a mercapto group was simultaneously introduced. Subsequently, the solvent was replaced with pure water, and buffering salts were added. Thereafter, added was oligonucleotide in which a mercapto group was introduced into one end and a methyloxycarbonyl group was introduced into the other end was added to attain 100 nM. The resulting mixture was allowed to stand for one hour so that the inorganic fluorescent nanoparticles were bound to oligonucleotide while allowing the mercapto group to form a sulfide bond. The state in which the above inorganic fluorescent nanoparticles are bound to oligonucleotide corresponds to a state in which a methyloxycarbonyl group is further bound to the end portion of the modifying group of Sample A. A fluorescent marker, prepared as above, was designated as Sample B.

Further, a fluorescent marker was produced in the same manner as above, except that inorganic fluorescent nanoparticles prepared via the HF etching method were replaced with those prepared via the anodic oxidation method. The resulting material was designated as Sample D.

(Introduction of DNA Base End Modifying Group: Sample E) Initially, based on the particle size, the inorganic fluorescent nanoparticles, prepared via the HF etching method, were divided into four portions, each of which exhibited an average particle diameter of 1.5 nm, 2.0 nm, 2.5 nm, or 3.0 nm, respectively. In each of them, SiO₂ of the uppermost surface was silanized and a mercapto group was introduced in the same manner as in the case of the preparation of Sample A. Subsequently, the solvent was replaced with pure water and buffering salts were added. Further added was an adenine base in which a mercapto group was introduced into one end was added to attain 100 nM. The resulting mixture was allowed to stand for one hour, and the adenine base was bound to the end via formation of sulfide among mercapto groups. In the same manner, inorganic fluorescent nanoparticles were prepared into which a modifying group which had a guanine base, a cytosine base, and a thymine base at the end was introduced. Sample E was prepared by mixing Si quantum dots in which the four modifying groups as well as the sizes differed.

(DNA Analysis Method) Inorganic fluorescent nanoparticles, to which the end of oligonucleotide had been bound, were placed in the well of a micro-array slide. DNA to be analyzed was dripped into the well, and subsequently, the resulting mixture was placed into a thermostat. The well interior was heated to several tens degree and under humidified conditions, incubated for one day. During incubation, when both of the oligonucleotide and the DNA to be detected, each of which has a corresponding base sequence, were present in the same well, these underwent hybridization reaction.

Subsequently, the well was washed with pure water and a fluorescent marker (being Sample A) bound to oligonucleotide, which had not undergone the hybridization, in the well was removed, whereby inorganic fluorescent nanoparticles, bound to a nucleotide which had undergone the hybridization reaction, remained in the well.

Subsequently, 430 nm excitation light was exposed to the specified well, and fluorescent address information was detected via a fluorescent microscope (produced by Olympus Optical Co., Ltd.), and detection accuracy was visually evaluated. The detection was evaluated as follows: A case in which fluorescence was significantly bright and a hybridization resulting map was clearly noticed was evaluated to be "good", while a case in which the fluorescent intensity was low and the above map was not so easily noticed was evaluated to be "poor" for detection. Further, a sample, which was separately incubated and was subjected to hybridization, was exposed to 400 nm excitation light employing FP-6500 produced by JASCO Corp. and the intensity of the resulting fluorescence was determined.

Measurement was carried out for Samples B, C, and D in the same manner as for above Sample A. Table 1 shows the results of the above fluorescent intensity and visual detection accuracy. Each of the fluorescent intensifies was represented by a relative value when the fluorescent intensity of Sample B was considered to be 100.

As is seen in Table 1, by introducing, into the end portion, a complementarily binding portion based on the present invention, the fluorescent intensity was increased and the detection accuracy for DNA was markedly increased.
Further, by blending Sample E with DNA to be detected in the well and by employing the mixture in the micro-array well for detection, it is possible to represent the base sequence of the DNA employing the different color sequence, whereby it is possible to collect DNA sequence information. Adenine base modified inorganic fluorescent nanoparticles were blue; guanine base modified inorganic fluorescent nanoparticles were bluish green; cytosine base modified inorganic fluorescent nanoparticles were green; and thymine modified inorganic fluorescent nanoparticles were red.

**Table 1**

| Sample | Production method of inorganic fluorescent nanoparticles | Type of modifying group | Fluorescent intensity: relative intensity when sample B was used as a standard (100) | Detection accuracy (visual) | Remarks |
|---|---|---|---|---|---|
| A | HF etching method | End: oligonucleotide | 150 | Good | Present invention |
| B | HF etching method | Intermediate position: oligonucleotide (same as that of sample A) | 100 | Resulting in poor detection | Comparative |
| C | Anodic oxidation method | End: oligonucleotide (one having different base sequence from sample A) | 160 | Good | Present invention |
| D | Anodic oxidation method | Intermediate position:oligonucleotide (one which was the same as sample C) | 98 | Resulting in poor detection | Comparative |

Example 2 (Introduction of Oligonucleotide End Modifying Group: Samples A2 and C2) The inorganic fluorescent nanoparticles prepared via the HF etching method which was employed in Example 1 were divided into two portions.

Initially, one portion was dispersed into 30% H₂O₂ for 10 minutes, whereby the surface of the crystals was hydroxidized. Subsequently, the solvent was replaced with toluene, and a mixture, prepared by blending mercaptopropyltriethoxysilane and aminopropyltriethoxysilane at a ratio of 4 : 1, was added in an amount of 2% of toluene. Subsequently, SiO₂ of the uppermost surface of the inorganic fluorescent nanoparticles was silanized over two hours, while a mercapto group and an amino group were introduced at a ratio of 4 : 1, in terms of surface area. After replacing the solvent with pure water, buffering salts were added and further, oligonucleotide, in which a mercapto group was introduced into one end, was added to attain 100 nM. followed by allowing it to stand for one hour, whereby the mercapto groups were allowed to form a sulfide bond, further followed by binding the inorganic fluorescent nanoparticles to the oligonucleotide.

The other portion was subjected to introduction of a mercapto group and an aldehyde group at a ratio of 4 : 1 in terms of surface area in the same manner as above, except that the above aminopropyltriethoxysilane was replaced with a silane coupling agent into which an aldehyde group was introduced, and further, oligonucleotide in which a mercapto gropup was introduced into the end was bound.

The above two types of inorganic fluorescent nanoparticles were blended at 30 °C for two hours. A resulting fluorescent marker was designated as Sample A2. Based on IR and molecular weight analysis, it was found that in Sample A2, an amino group and an aldehyde group reacted with each other to form a link, and an average of 6 nanoparticles of the inorganic fluorescent nanoparticles aggregated to form a cluster.

Further, a fluorescent marker was produced in the same manner as described above, except that inorganic fluorescent nanoparticles prepared by the anodic oxidation method were replaced with those prepared by the HF etching method. The resulting martial was designated as Sample C2.

(Introduction of modifying group which places oligonucleotide at the intermediate position: Sample B2) Initially, inorganic fluorescent nanoparticles prepared via the HF etching method were dispersed into 30% H₂O₂ for 10 minutes, whereby the surface of crystals was hydroxidized. Subsequently, the solvent was replaced with toluene, and mercaptoethyltriethoxysilane in an amount of 2% of toluene was added. Subsequently, SiO₂ of the uppermost surface of the inorganic fluorescent nanoparticles was silanized over two hours, while a mercapto group was introduced. After replacing the solvent with pure water, buffering salts were added and further, oligonucleotide in which a mercapto group was introduced into one end was added to reach 100 nM, followed by allowing it to stand for one hour, whereby the mercapto groups were allowed to form a sulfide bond, further followed by binding the inorganic fluorescent nanoparticles to the oligonucleotide. The fluorescent marker prepared as above was designated as Sample B2. Resulting Sample B2 was in the state in which only a living organism binding portion in the present invention was introduced.

A fluorescent marker was produced in the same manner as above, except that inorganic fluorescent nanoparticles prepared via the HF etching method was replaced with those prepared via the anodic oxidation method. The resulting material was designated as Sample D2. (Introduction of a linked modifying group which have oligonucleotide at the intermediate position: Sample E2) The inorganic fluorescent nanoparticles prepared via the HF etching method which was employed in Example 1 were divided into two portions.

Initially, one portion was dispersed into 30% H₂O₂ for 10 minutes, whereby the surface of the crystals was hydroxidized. Subsequently, the solvent was replaced with toluene, and a mixture, prepared by blending mercaptopropyltriethoxysilane and aminopropyltriethoxysilane at a ratio of 4 : 1, was added in an amount of 2% of toluene. Subsequently, SiO₂ of the uppermost surface of the inorganic fluorescent nanoparticles was silanized over two hours, while a mercapto group and an amino group were introduced at a ratio of 4 : 1, in terms of surface area. After replacing the solvent with pure water, buffering salts were added and further, oligonucleotide, in which a mercapto group was introduced into one end and a methyloxycarbonyl group was introduced on the other end, was added to attain 100 nM. followed by allowing it to stand for one hour, whereby the mercapto groups were allowed to form a sulfide bond, further followed by binding the inorganic fluorescent nanoparticles to the oligonucleotide.

The other portion was subjected to introduction of a mercapto group and an aldehyde group at a ratio of 4 : 1 in terms of surface area in the same manner as above, except that the above aminopropyltriethoxysilane was replaced with a silane coupling agent into which an aldehyde group was introduced. And further, oligonucleotide in which a mercapto gropup was introduced into the end and a methyloxycarbonyl group was introduced on the other end, was added to attain 100 nM. followed by allowing it to stand for one hour, whereby the mercapto groups were allowed to form a sulfide bond, further followed by binding the inorganic fluorescent nanoparticles to the oligonucleotide.

The above two types of inorganic fluorescent nanoparticles were blended at 30 °C for two hours. A resulting fluorescent marker was designated as Sample E2. Based on IR and molecular weight analysis, it was found that in Sample E2, an amino group and an aldehyde group reacted with each other to form a link, and an average of 6 nanoparticles of the inorganic fluorescent nanoparticles aggregated to form a cluster.

Samples A2 - E2, prepared via the above methods, were subjected to DNA analysis in the same manner as Example 1. Table 2 shows these results related to fluorescent intensity and visual detection accuracy. Each of the fluorescent intensities was represented by a relative value when the fluorescent intensity of Sample B2 was considered to be 100.

As can be seen in Table 2, by employing the fluorescent marker into which the link forming modifying group according to the present invention was introduced, fluorescent intensity was increased and detection accuracy for DNA was markedly enhanced.

**Table 2**

| Sample | Production method of inorganic fluorescent nanoparticles | Type of modifying group | Fluorescent intensity: relative intensity when sample B2 was used as a standard (100) | Detection accuracy (visual) | Remarks |
|---|---|---|---|---|---|
| A2 | HF etching method | End: oligonucleotide | 190 | Good | Present |
| B2 | HF etching method | Intermediate position: oligonucleotide (same as that of sample A2) | 100 | Resulting in poor detection | Comparative |
| C2 | Anodic oxidation method | End: oligonucleotide (one having different base sequence from sample A2) | 180 | Good | Present invention |
| D2 | Anodic oxidation method | Intermediate position: oligonucleotide (one which was the same as sample C2) | 98 | Resulting in poor detection | Comparative |
| E2 | HF etching method | Intermediate position: oligonucleotide (same as that of sample A2) and having a link forming modifying group | 130 | Good | Present invention |

## Claims

1. A fluorescent marker for a living organism comprising inorganic fluorescent nanoparticles having on a surface of the nanoparticles:
a modifying group containing a living organism binding portion capable of specifically binding to a living organism; and
a link forming modifying group which combines the inorganic fluorescent nanoparticles together.

2. The fluorescent marker for a living organism of claim 1, wherein a surface covering ratio of the link forming modifying groups on the surfaces of the inorganic fluorescent nanoparticles is 10-50%.

3. The fluorescent marker for a living organism of claims 1 or 2, wherein the inorganic fluorescent nanoparticle is a spherical particle having an average particle diameter of 1-10 nm.

4. The fluorescent marker for a living organism of any one of claims 1 to 3, wherein the inorganic fluorescent nanoparticle has a core-shell structure comprising:
a core of a semiconductor; and
a shell having a different composition from the core.

5. The fluorescent marker for a living organism of claim 4, wherein the inorganic fluorescent nanoparticle has:
the core of Si; and
the shell of SiO₂.

6. The fluorescent marker for a living organism of any one of claims 1 to 5, wherein the link forming modifying group combines the inorganic fluorescent nanoparticles together through a hydrogen bond, an ionic bond, a covalent bond or a Van der Waals' force.

7. The fluorescent marker for a living organism of any one of claims 1 - 6, wherein the inorganic fluorescent nanoparticles are in a form of a cluster having a particle number of 2-20.

8. A method of fluorescent marking for a living organism using the fluorescent marker of any one of claims 1 to 7, the method comprising the step of:
binding the living organism with the inorganic fluorescent nanoparticle through the reactive portion of the modifying group capable of specifically binding to the living organism.
